# EUROPEAN PATENT APPLICATION

(11) **EP 1 118 612 A1**
(43) Date of publication of application: **25.07.2001**
(21) Application number: 00101165.9
(22) Date of filing: 21.01.2000
(51) Int. Cl.: C07D 295/22, C07D 295/18, A61K 31/495, A61P 25/28

(54) **Piperazine derivatives possesing nootropic activity**

(71) Applicant: Universita Degli Studi di Firenze, 50121 Florence (IT)
(72) Inventor: Bartolini, Alessandro, 52025 Montevarchi (AREZZO) (IT); Bellucci, Cristina, 50141 Firenze (IT); Galeotti, Nicoletta, 56038 Ponsacco (PISA) (IT); Ghelardini, Carla, 51030 Pistoia (IT); Gualtieri, Fulvio, 50018 Scandicci (SIENA) (IT); Manetti, Dina, 50028 Tavarnelle Val di Pesa (FIRENZE) (IT)
(74) Representative: Gervasi, Gemma, Dr.

(57) **Abstract**

Piperazine derivatives with general structure (I) are described. There: 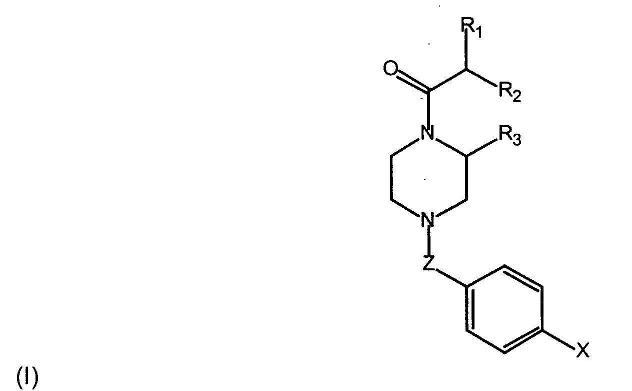
R1 can be H or C1-5 alkyl;
R2 and R3 are both H or linked together to form a bridge -CH₂-;
X can be H, F, OCH₃, Cl, NO₂, NH₂, CH₃;
Z can be CO, SO₂.

The compounds described above are endowed with an interesting nootropic activity that makes them suitable to treat neurodegenerative diseases such as Alzheimer's disease.

## Description

### Field of the invention

The present invention refers to a new class of piperazine derivatives having general formula (I) 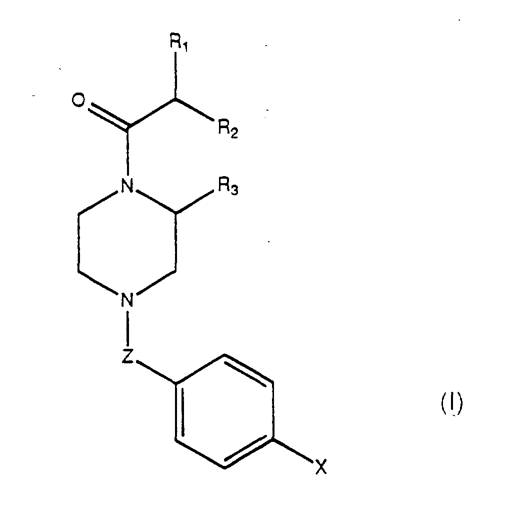 wherein:
R1 is chosen in the group consisting of H or C1-5 alkyl;
R2 and R3 are both H or are linked together to form a bridge -CH₂-;
X is chosen in the group consisting of H, F, OCH₃, Cl, NO₂, NH₂ and CH₃;
Z is chosen in the group consisting of CO and SO₂.

The claimed compounds are endowed with an interesting nootropic activity that makes them suitable to treat neurodegenerative diseases such as Alzheimer's disease.

The present invention refers also to the use of the above said compounds of formula (I) to prepare pharmaceutical compositions as well as to the pharmaceutical compositions containing them.

### Prior art

After the discovery, some thirty years ago, of the nootropic properties of piracetam that made possible to improve cognitive functions of subjects treated with this drug, several related molecules have been synthesised and studied and few of them are presently used in the medical practice (under the name of nootropic compounds).

Although, up today, their mechanism of action has not been clarified, such compounds are indeed able to revert amnesia induced by scopolamine, electroconvulsive shock, hypoxia and so on, possibly by increasing neuronal sensitivity toward stimulation. This class of compounds is characterised by a very low toxicity as they lack the side effects that are recorded when analeptics or psychostimulants are used.

Recent reports [Parnetti et al. "Cognitive enhancement therapy for Alzheimer's disease" *Drugs 53:* 752-768 (1997)]have shown that piracetam-like compounds

definitely improve cognitive functions of patient affected by mild or moderate Alzheimer's disease and are very well tolerated.

However, despite the success reached in the field, the need for new, safer and more potent drugs to treat neurodegenerative diseases does not need to be stressed.

### Description of the invention

The claimed invention refers to new compounds of general formula (I) as above defined, endowed with nootropic activity and useful to treat neurodegenerative pathologies connected with ageing, such as Alzheimer's disease.

It has been surprisingly found that the presence of an acyl- or solfonyl-aromatic group directly linked to the piperazine nitrogen allows an easier crossing of the blood brain barrier without impairing, and rather improving; the properties of the compounds.

According to the invention, preferred R₁ substituents are: methyl, ethyl, n-propyl, and n-butyl; as far as X is concerned, groups like NO₂, OCH₃, Cl are preferred.

According to the general formula (I), some of the compounds represented by that structure may present stereogenic centres. They are usually obtained as racemic mixtures but the present invention refers also to the corresponding enantiomers.

The products under the present invention can be prepared by known procedure of the art.

To exemplify, formula (I) derivatives, where R₁ = H and R₂ and R₃ are linked into a CH₂ bridge, can be prepared (according to scheme 1) starting from 1,4-dibenzyl-2-piperazincarboxaldehyde II [obtained as described by Manetti et al. - Med.

Chem. Res. 7: 301-12 (1997)] that is reacted with the anion obtained from diethyl [1-(methoxycarbonyl) methyl]phosphonate to give compound III. This intermediate is reduced to the corresponding debenzylated alkane that, in turn, spontaneously cyclises to compound IV. This compound is eventually N-substituted, using suitable acyl- and solfonyl-chlorides, to afford final compounds 1, 2, 9, and 10 (see Table I).

Accordingly, compounds of formula (I) where R1 is CH₃ and R₂ and R₃ are linked into a CH₂ bridge, can be prepared either as diastereoisomeric mixtures (a+b) or as pure diastereoisomers (a and b) by acylation with the proper acyl- or solfonyl-chloride (see Scheme 2). In the first case, the starting material is the mixture of diastereoisomers V(a+b), in the second case the starting material are the pure diastereoisomers Va and Vb that can be obtained by debenzylation of the two pure diastereoisomers of the corresponding benzylderivative already described [Manetti et al. - Med. Chem. Res. 7: 301-12 (1997)].

Finally, compounds (I) where R2 and R3 are both H, can be obtained starting from N-benzylpiperazine VI (commercially available) by acylation with the proper acylchloride to give compounds with general structure VII, that after removal of the benzyl group, give the acylderivatives of general formula VIII. Reaction with the suitable aromatic acyl- or solfonyl-chloride gives the final compounds (see Scheme 3a). When, in compounds with general structure VIII, R1 = H the final products are obtained by direct acylation of commercially available N-acetylpiperazine IX (see Scheme 3b).

The following examples illustrate the synthesis of some of the derivatives claimed in the invention.

Methyl 1,4-dibenzyl-2-piperazinyl-3-acrylate (III)

Diethyl[1-(methoxycarbonyl)methyl]phosphonate (0.39 g, 1.85 mmol) is added to NaH (45 mg, 1.85 mmol, washed three times with anhydrous hexane) and the mixture suspended in anhydrous dimethoxyhexane (DME, 4.6 ml). After one hour, a DME solution (5 ml) of 1,4-dibenzyl-2-piperazincarboxaldehyde (0.54 g, 1.85 mmol) is added. After one hour at room temperature, the reaction mixture is washed with water and extracted with Et₂O. Evaporation of the solvent leaves a residue that is purified by SiO₂ column chromatography (eluent: cyclohexane/ethylacetate 50:50) to afford the title compound (III) as a oily mixture of isomers.

(±) 1,4-diazabicyclo[4.3.0]nonan-9-one (IV)

The compound obtained as described above (III) (0.30 g, 1.10 mmol) is hydrogenated over Pd/C 10% (0.19 g) in abs. EtOH at 50 psi for 8 hours. After filtration of the catalyst, the solvent is removed under vacuum and the residue purified by SiO₂ column chromatography (eluent: CHCl₃/MeOH/hexane 72:8:20) to give the title compound (IV) as an oil.

Exo (Va) and Endo (Vb) (±) 8-methyl-1,4-diazabicyclo[4.3.0]nonan-9-one

Exo , or endo (±) 4-benzyl-8-methyl-1,4-diazabicyclo[4.3.0]nonan-9-one [obtained according to Manetti et al. - Med. Chem. Res. 7: 301-12 (1997)] ) is hydrogenated over Pd/C 10% (0.06 g) in abs. EtOH at 47 psi for 12 hours. After filtration of the catalyst the solvent is removed under vacuum to give pure title compounds (Va and Vb) as oils.

1-Benzyl-4-propionylpiperazine (VII; R₁ = CH₃)

Propionylchloride(0.09 ml, 1 mmol) and N(C₂H₅)₃ (0.2 ml, 1,5 mmol) are added to N-benzylpiperazine (0.17 ml, 1 mmol). After 2 h at room temperature 50 ml of Et₂O are added and the solution washed with H₂O. After drying the solvent is removed and the residue purified by SiO₂ flash chromatography (eluent: petrol eter/CH₂Cl₂/(C₂H₅)₂/abs. EtOH /NH₄OH 900:360:360:180:9.9)to give title compound VII.

In the same way compounds where R₁ = C₂H₅, C₃H₇, C₄H₉, C₅H₁₁, were obtained, using the appropriate acylchloride.

N-propionilpiperazina (VIII; R₁ = CH₃)

1-Benzyl-4-propionylpiperazine (0.22 g, 1 mmol) is hydrogenated over Pd/C 10% (0.10 g) in abs. EtOH at 47 psi for 12 hours. After filtration of the catalyst, the solvent is removed under vacuum to give nearly pure title compounds (VIII) that is used as such in the following reaction.

In the same way, starting from the appropriate 1-benzyl-4-acylpiperazine, compounds where R₁ = C₂H₅, C₃H₇, C₄Hg, C₅H₁₁, were obtained.

Acyl- and solfonyl derivatives synthesis

Amyl- or slovenly chloride and N(C₂H₅)₃ (1.5 mil) are added to the appropriate amine (IV, V, Ver, VB, VIII, IX) dissolved in a few ml of CH₃CN. After two hours at room temperature, 50 ml of Et₂O are added and the solution washed with H₂O.

The solvent is removed under vacuum, the residue purified through flash chromatography and finally crystallised from the suitable solvent.

Following the way described above, the following compounds have been synthesised. For each the solvent used in flash chromatography, the crystallisation solvent and melting point are reported (see also Tables 1, 2, 3 and 4):
4-(4-fluorobenzoyl)-1,4-diazabicyclo[4.3.0]nonan-9-one (eluent: CHCl₃/MeOH/90:10; m.p.: 142-3 °C, from petrol ether) (1).
4-(4-fluorobenzensolfonyl)-1,4-diazabicyclo[4.3.0]nonan-9-one (eluent: CHCl₃/MeOH/ 90:10; m.p.: 157-9 °C, from petrol ether) (2).
Endo/exo 8-methyl-4-(4-fluorobenzoyl)-1,4-diazabicyclo[4.3.0]nonan-9-one (eluent: CHCl₃/MeOH/ 90:10; m.p.: 130-1 °C, from petrol ether) (3 a+b).
Exo (±) 8-methyl-4-(4-fluorobenzoyl)-1,4-diazabicyclo[4.3.0]nonan-9-one (eluent: CHCl₃/MeOH/ 90:10; m.p.: 142-3 °C, from petrol ether) (3a).
Endo (±) 8-methyl-4-(4-fluorobenzoyl)-1,4-diazabicyclo[4.3.0]nonan-9-one (eluent: CHCl₃/MeOH/ 90:10; m.p.: 135-7 °C, from petrol ether) (3b).
Endo/exo 8-methyl-4-(4-fluorobenzensolfonyl)-1,4-diazabicyclo[4.3.0]nonan-9-one (eluent: CHCl₃/MeOH/ 97:3; m.p.: 97-99 °C, from petrol ether) (4 a+b).
Exo 8-methyl-4-(4-fluorobenzensolfonyl)-1,4-diazabicyclo[4.3.0]nonan-9-one (eluent: CHCl₃/MeOH/ 97:3; m.p.: 97-99 °C, from petrol ether) (4a).
Endo 8-methyl-4-(4-fluorobenzensolfonyl)-1,4-diazabicyclo[4.3.0]nonan-9-one (eluent: CHCl₃/MeOH/ 97:3; m.p.: 125-7 °C, from petrol ether) (4b).
4-Acetyl-1-(4-fluorobenzoyl)piperazine (eluent: CHCl₃/EtOH an./petrol ether/NH₄OH 360:65:60:8; m.p.: 50-2°C from petrol ether) (5).
4-Acetyl-1-(4-fluorobenzensolfonyl)piperazine (eluent: CHCl₃/EtOH an./petrol ether/NH₄OH 360:65:60:8; m.p.: 100-2 °C, from petrol ether) (6).
4-Propionyl-1-(4-fluorobenzoyl)piperazine (eluent: CHCl₃/EtOH an./ petrol ether /NH₄OH 360:65:60:8; m.p.: 90-1 °C , from petrol ether) (7).
4-Propionyl-1-(4-fluorobenzensolfonyl)piperazine (eluent: CHCl₃/EtOH an./ petrol ether/NH₄OH 360:65:60:8; m.p.: 79-80 °C, from petrol ether) (8).
4-Benzoyl-1,4-diazabicyclo[4.3.0]nonan-9-one (9)
4-Benzenesolfonyl-1,4-diazabicyclo[4.3.0]nonan-9-one (10)
Exo ed Endo 8-methyl-4-benzoyl-1,4-diazabicyclo[4.3.0]nonan-9-one (11a; 11b)
Exo ed Endo 8-methyl-4-benzensolfonyl-1,4-diazabicyclo[4.3.0] nonan-9-one (12a; 12b)
4-Acetyl-1-benzoylpiperazine (13)
4-Acetyl-1-benzensolfonylpiperazine (14)
4-Propionyl-1-benzoylpiperazine (15)
4-Propionyl-1 -benzensolfonylpiperazine(16)
4-Butanoyl-1-(4-fluorobenzoyl)piperazine (17)
4-Butanoyl-1-(4-fluorobenzensolfonyl)piperazine (18)
4-Pentanoyl-1-(4-fluorobenzensolfonyl)piperazine (19)
4-Hexanoyl-1-(4-fluorobenzensolfonyl)piperazine (20)
4-(4-methylbenzensolfonyl)-1,4-diazabicyclo[4.3.0]nonan-9-one (21)
4-(4-nitrobenzensolfonyl)-1,4-diazabicyclo[4.3.0]nonan-9-one (22)
4-(4-chlorobenzensolfonyl)-1,4-diazabicyclo[4.3.0]nonan-9-one (23)
4-(4-methoxybenzensolfonyl)-1,4-diazabicyclo[4.3.0]nonan-9-one (24)
4-(4-methylbenzoyl)-1,4-diazabicyclo[4.3.0]nonan-9-one (25)
4-Propionyl-1-(4-methylbenzensolfonyl)piperazine (26)
4-Propionyl-1-(4-methoxyylbenzensolfonyl)piperazine (27)
4-Propionyl-1-(4-chlorobenzensolfonyl)piperazine (28)
4-Propionyl-1-(4-nitrobenzensolfonyl)piperazine (29)
4-Propionyl-1-(4-methylbenzoyl)piperazine (30)
4-Propionyl-1-(4-methoxybenzoyl)piperazine (31)
4-Propionyl-1-(4-chlorobenzoyl)piperazine (32)
4-Propionyl-1-(4-nitrobenzoyl)piperazine (33)

### Pharmacology

### Evaluation of cognitive activity (Passive-Avoidance test)

A slightly modified step-through method described by Jarvik and [Psycol. Rep. 21:221-4 (1967)] has been used to evaluate nootropic activity of claimed compounds. The apparatus consists of a two-compartment acrylic box with a lighted compartment connected to the darkened one by a guillotine door. In the original method, mice received a punishing electrical shock as soon as they entered the dark compartment, while in our modified method, after entry into the dark compartment, mice receive a non-painful punishment consisting of a fall (from 40 cm ) into a cold water bath (10°C) . For this purpose the dark chamber was constructed with a pitfall floor. The latency times for entering the dark compartment were measured in the training test (first day) and after 24 h in the retention test (second day). Mice that did not enter after 60 s latency were excluded from the experiment. For memory disruption, mice were i.p. injected with the amnesic drugs (scopolamine, mecamylamine, baclofen and clonidine). All investigated drugs were injected 20 min before the training session, while amnesic drugs were injected immediately after termination of the training session. The maximum entry latency allowed in the retention session was 120 s. The memory degree of received punishment (fall into cold water) was expressed as the increase in seconds between training and retention latencies (see Table 5).

As it can be seen from the table, all compounds claimed are able to revert induced amnesia at doses that are always lower (except in one case) than that of piracetam used as a reference drug.

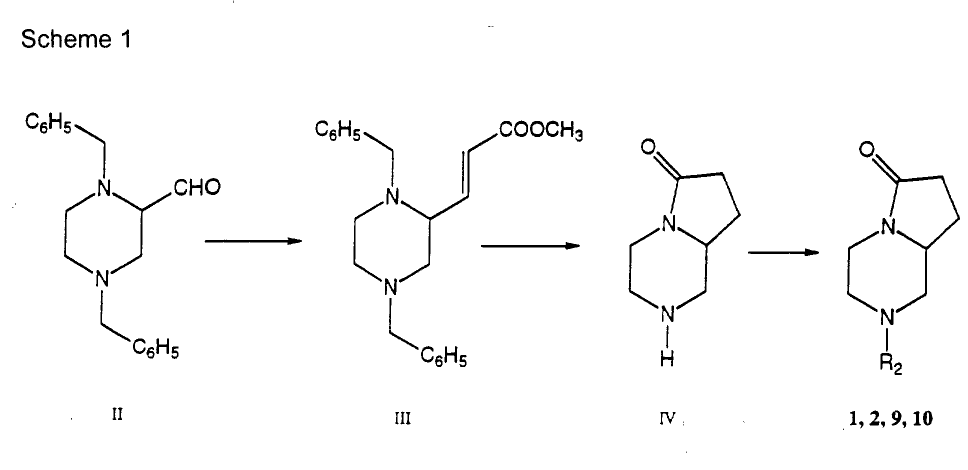

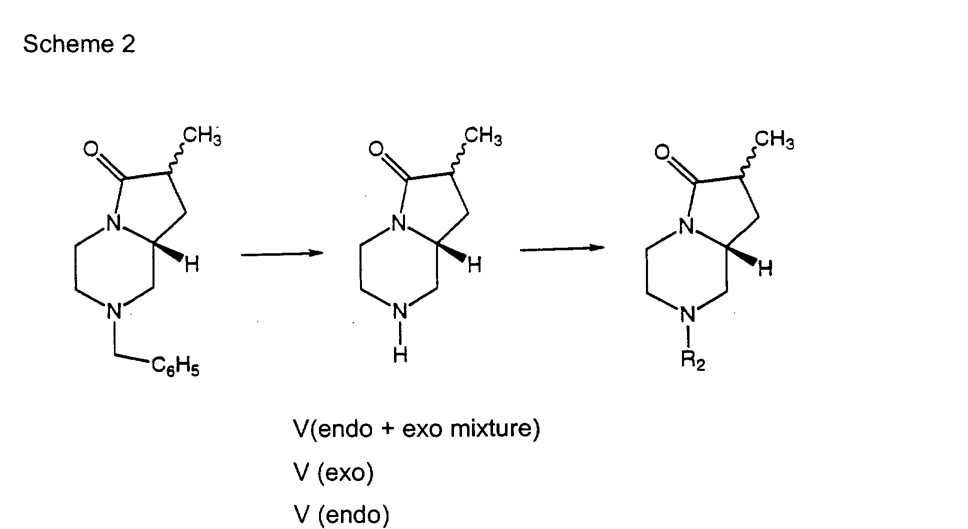

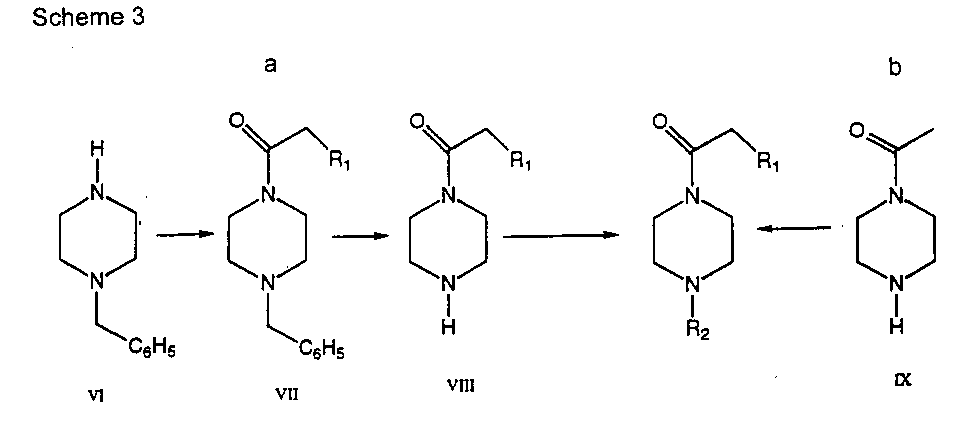

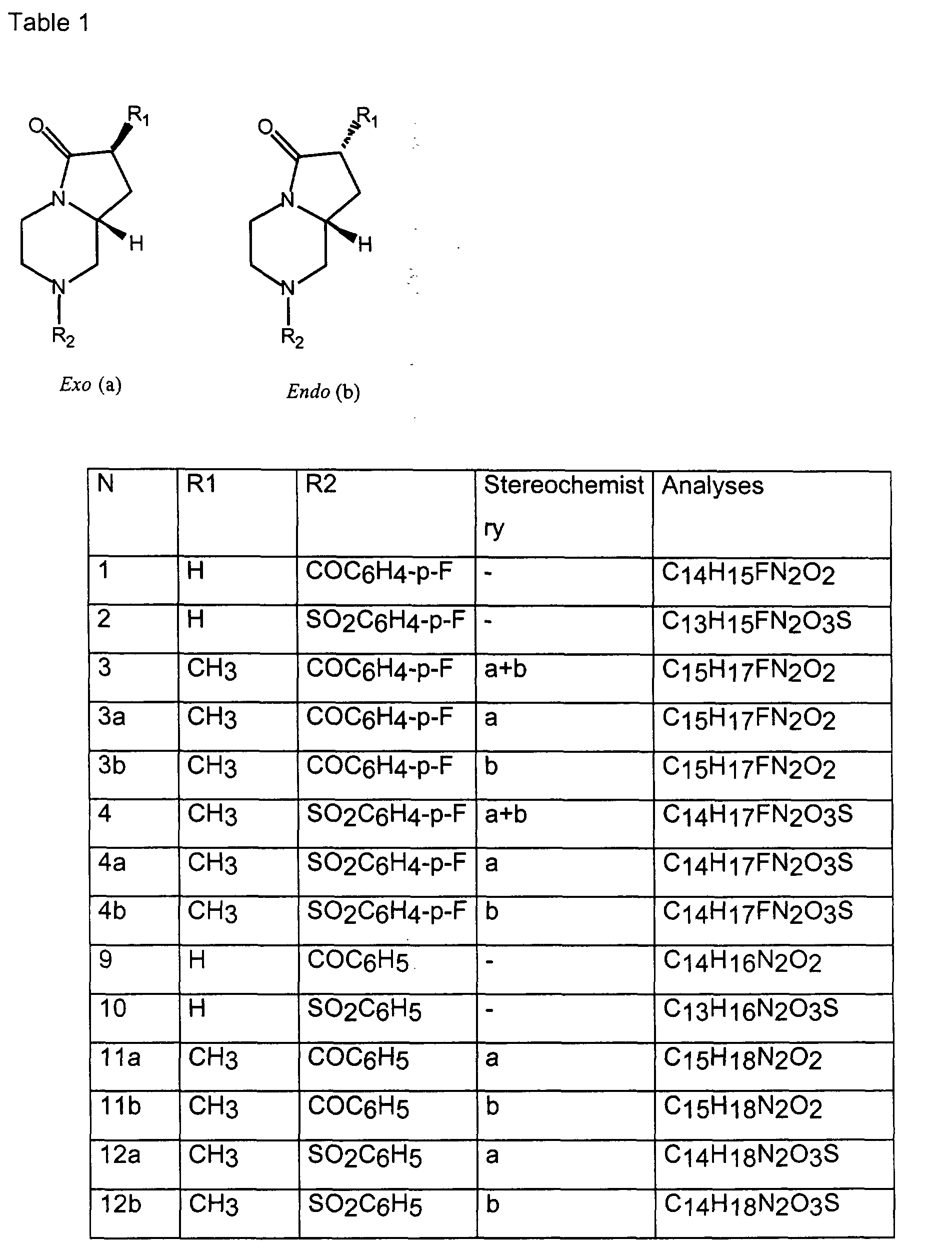

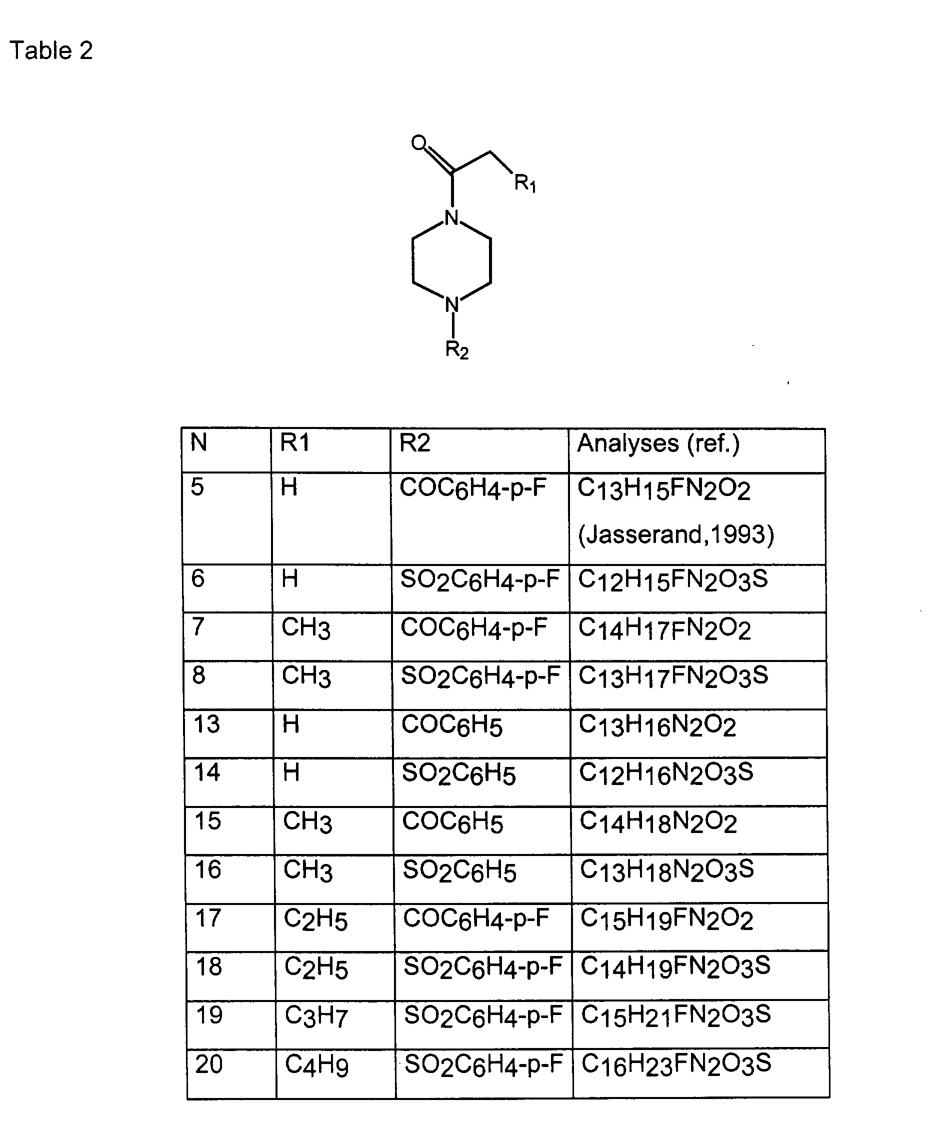

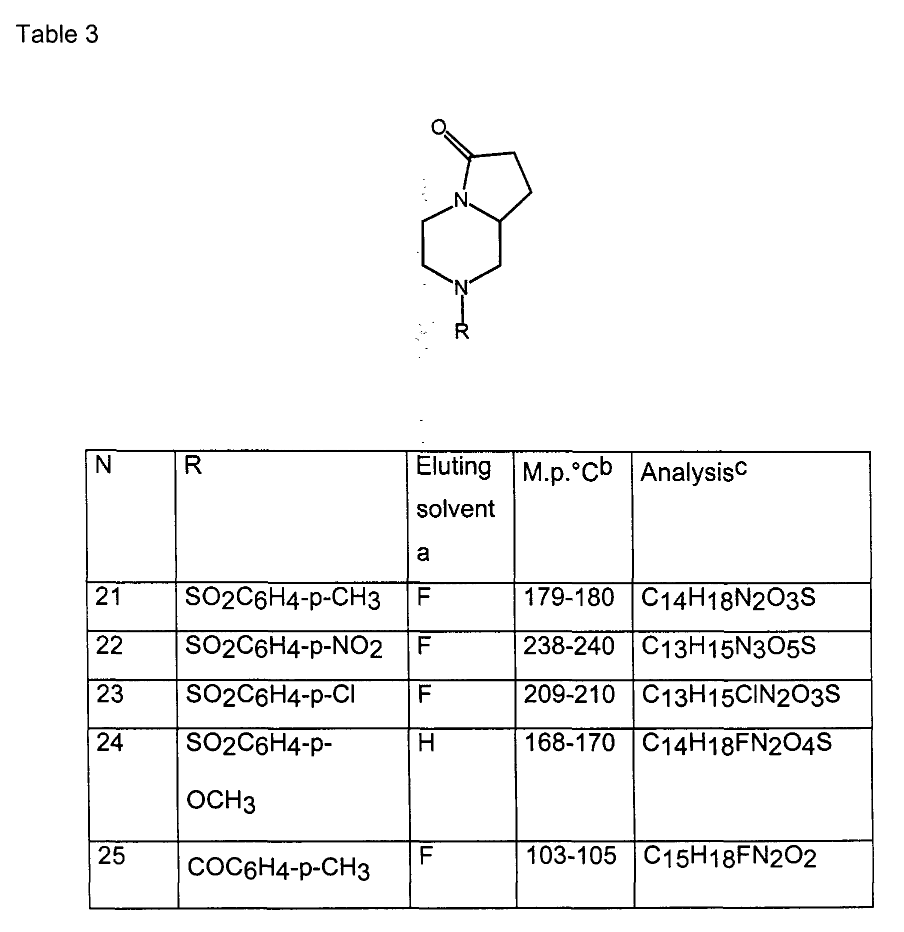

Eluting system for column chromatography: A = CHCl₃/ MeOH, 90:10; B = CHCl₃/ MeOH, 97:3; C = CHCl₃/ an.Et OH/Petrol ether/NH₄OH 360:65:60:8; D = cyclohexane/ ethyl acetate, 50:50; E = CHCl₃/ MeOH, 95:5; F = CHCl₃/ MeOH/hexane, 65:5:30; G = CH₂Cl₂/ MeOH, 98:2; H = CH₂Cl₂/ MeOH, 95:5; b) After crystallization from petrol ether; c) ¹H-NMR and IR spectra are in accord with the proposed structures.

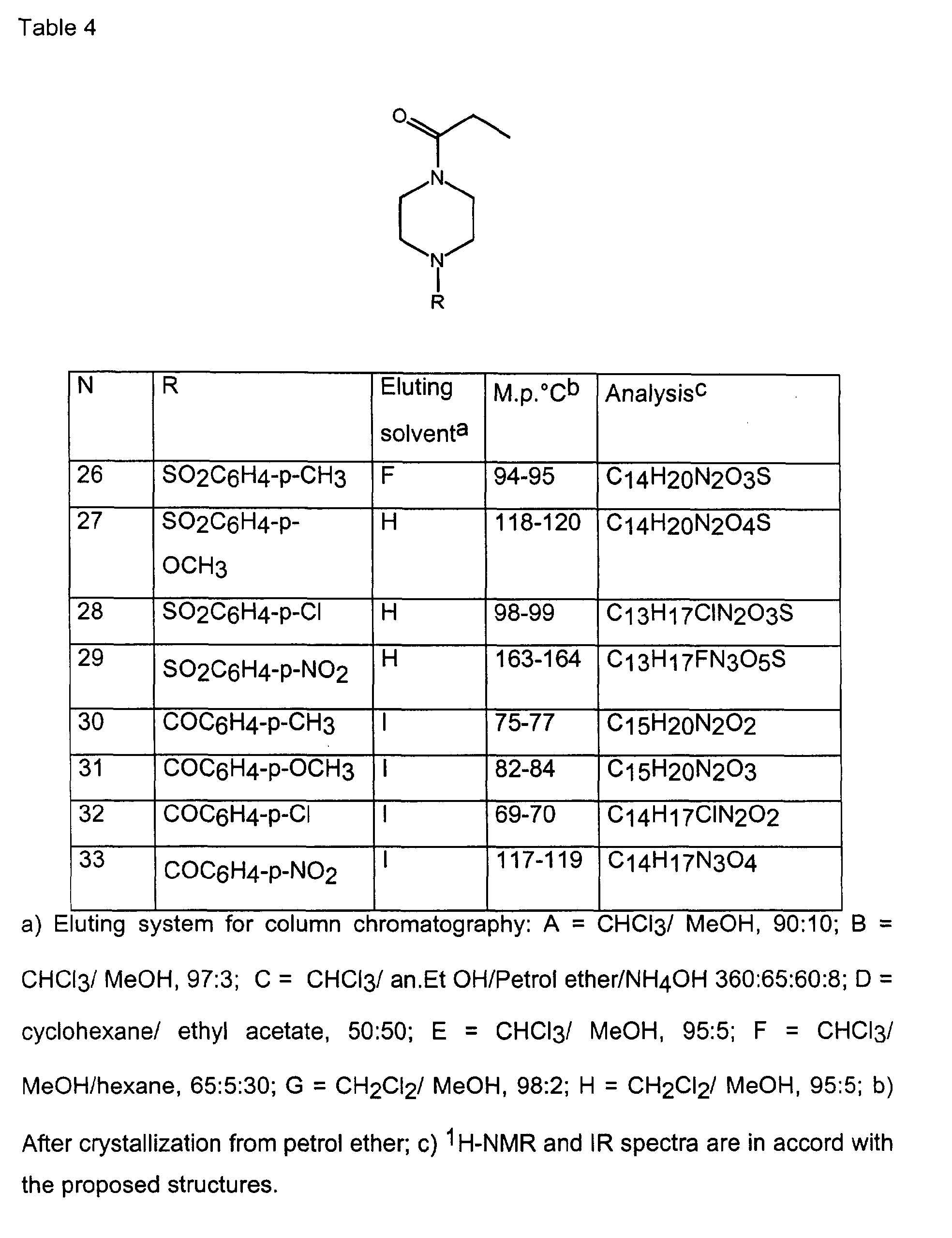

**Table 5**

| Nootropic activity of compounds 1, 2, 3a, 3b, 4a, 4b, 5, 6, 7, and 8 compared to piracetam in mouse passive avoidance test. | | | | | | |
|---|---|---|---|---|---|---|
| Treatment | | | 1^{st} Day | 2^{nd} Day | Δ | Mice |
| Saline | | + Saline | 15.3±3.8 | 101.4±10.6 | 86.1 | 27 |
| Saline | | + Scopolamine | 13.5±5.3 | 53.5±10.3° | 40.0 | 45 |
| 1 | 0.1 mg kg ⁻¹ | + Scopolamine | 18.1±3.9 | 100.3±10.4^{*} | 82.2 | 13 |
| 2 | 0.001 mg kg ⁻¹ + | Scopolamine | 19.7±5.8 | 104.4±10.6^{*} | 93.8 | 12 |
| 3a | 0.1 mg kg ⁻¹ | + Scopolamine | 21.8±4.8 | 107.4±10.7^{*} | 85.6 | 9 |
| 3b | 0.1 mg kg ⁻¹ | + Scopolamine | 12.5±5.9 | 104.0±8.6^{*} | 80.0 | 9 |
| 4a | 0.1 mg kg ⁻¹ | + Scopolamine | 16.9±6.1 | 94.9±11.2^{*} | 78.0 | 14 |
| 4b | 0.1 mg kg ⁻¹ | + Scopolamine | 21.9±4.7 | 118.4±10.2 | 96.5 | 11 |
| 5 | 10 mg kg ⁻¹ | + Scopolamine | 17.5±6.1 | 91.1±9.5^{*} | 72.6 | 12 |
| 6 | 0.01 mg kg ⁻¹ | + Scopolamine | 19.8±4.1 | 99.0±18.3^{*} | 79.2 | 10 |
| 7 | 30 mg kg ⁻¹ | + Scopolamine | 11.9±5.6 | 92.4±10.1^{*} | 80.5 | 10 |
| 8 | 0.01 mg kg ⁻¹ | + Scopolamine | 15.9±3.2 | 83.8±9.2^{*} | 67.9 | 26 |
| Piracetam | 30 mg kg ⁻¹ | + Scopolamine | 17.6±3.6 | 108.8±10.4^{*} | 91.2 | 34 |
| Saline | | + Baclofen | 17.9±4.0 | 68.4±7.5° | 50.5 | 29 |
| 5 | 1.0 mg kg ⁻¹ | + Baclofen | 11.4±5.2 | 104.6±10.3^{*} | 93.2 | 9 |
| 6 | 0.1 mg kg ⁻¹ | + Baclofen | 11.7±4.1 | 92.1±7.9^{*} | 80.4 | 9 |
| 7 | 30 mg kg ⁻¹ | + Baclofen | 20.6±4.9 | 107.1±11.4^{*} | 86.8 | 10 |
| 8 | 0.01 mg kg ⁻¹ | + Baclofen | 16.3±3.3 | 103.3±13.9^{*} | 86.8 | 9 |
| Piracetam | 30 mg kg ⁻¹ | + Baclofen | 20.9±4.5 | 96.5±11.7^{*} | 75.6 | 12 |
| Saline | | + Mecamilamine | 17.3±4.1 | 61.4±9.3° | 44.1 | 20 |
| 5 | 20 mg kg ⁻¹ | + Mecamilamine | 17.0±2.7 | 108.7±3.5^{*} | 91.7 | 8 |
| 6 | 1 mg kg ⁻¹ | + Mecamilamine | 18.1±3.8 | 109.7±13.6^{*} | 91.6 | 7 |
| 8 | 10 mg kg ⁻¹ | + Mecamilamine | 16.5±2.7 | 106.7±10.2^{*} | 90.2 | 10 |
| Piracetam | 30 mg kg ⁻¹ | + Mecamilamine | 16.7±4.8 | 115.0±12.9^{*} | 93.3 | 15 |
| Saline | | + Clonidine | 15.9±5.1 | 46.6±8.5° | 30.7 | 11 |
| 5 | 1.0 mg kg ⁻¹ | + Clonidine | 8.0±4.1 | 96.1±10.7^{*} | 88.1 | 11 |
| 6 | 0.1 mg kg ⁻¹ | + Clonidine | 14.7±3.7 | 92.4±8.1^{*} | 77.4 | 11 |
| Piracetam | 30 mg kg ⁻¹ | + Clonidine | 11.6±3.7 | 79.3±8.5 | 66.7 | 14 |

Doses: Scopolamine :1.5 mg kg ⁻¹ i.p.; Baclophen:2 mg kg ⁻¹ i.p.;
Mecamilamine: 20 mg kg ⁻¹ i.p.; Clonidine: 0.125 mg kg ⁻¹ i.p..
°P < 0.01 compared to mice treated with saline; *P < 0.01 compared to mice treated with the amnesing agent.

## Claims

1. Piperazine derivatives having general formula (I) 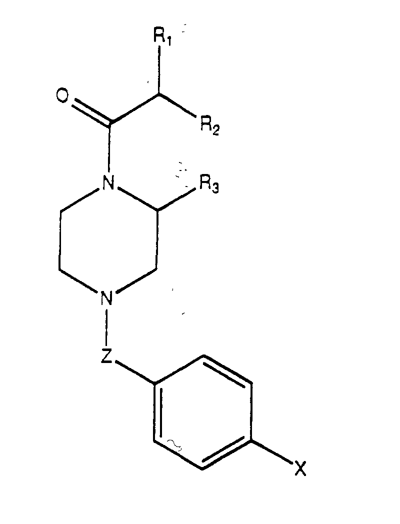 wherein:
R1 is chosen in the group consisting of H or C1-4 alkyl;
R2 and R3 are both H or are linked together to form a bridge -CH₂-;
X is chosen in the group consisting of H, F, OCH₃, Cl, NO₂, NH₂ and CH₃;
Z is chosen in the group consisting of CO and SO₂.

2. Derivatives according to claim 1 wherein:
R1 is chosen in the group consisting of: H, methyl, ethyl, n-propyl, n-butyl;
X is chosen in the group consisting of: NO₂, OCH₃, Cl.
Z, R2 and R3 are defined as above.

3. Derivatives according to claim 2 represented by:
4-(4-fluorobenzoyl)-1,4-diazabicyclo[4.3.0]nonan-9-one
4-(4-fluorobenzensolfonyl)-1,4-diazabicyiclo[4.3.0]nonan-9-one
Endo/exo 8-methyl-4-(4-fluorobenzoyl)-1,4-diazabicyclo[4.3.0]nonan-9-one
Exo (±) 8-methyl-4-(4-fluorobenzoyl)-1,4-diazabicyclo[4.3.0]nonan-9-one
Endo (±) 8-methyl-4-(4-fluorobenzoyl)-1,4-diazabicyclo[4.3.0]nonan-9-one
Endo/exo 8-methyl-4-(4-fluorobenzensolfonyl)-1,4-diazabicyclo[4.3.0]nonan-9-one
Exo 8-methyl-4-(4-fluorobenzensolfonyl)-1,4-diazabicyclo[4.3.0]nonan-9-one
Endo 8-methyl-4-(4-fluorobenzensolfonyl)-1,4-diazabicyclo[4.3.0]nonan-9-one
4-Acetyl-1-(4-fluorobenzoyl)piperazine
4-Acetyl-1-(4-fluorobenzensolfonyl)piperazine
4-Propionyl-1-(4-fluorobenzoyl)piperazine
4-Propionil-1-(4-fluorobenzensolfonil)piperazina
4-Benzoyl-1,4-diazabicyclo[4.3.0]nonan-9-one (9)
4-Benzenesolfonyl-1,4-diazabicyiclo[4.3.0]nonan-9-one (10)
Exo and Endo 8-methyl-4-benzoyl-1,4-diazabicyclo[4.3.0]nonan-9-one (11a; 11b)
Exo ed Endo 8-methyl-4-benzensolfonyl-1,4-diazabicyiclo[4.3.0]nonan-9-one (12a; 12b)
4-Acetyl-1-benzoylpiperazine (13)
4-Acetyl-1-benzensolfonylpiperazine(14)
4-Propionyl-1-benzoylpiperazine (15)
4-Propionyl-1-benzensolfonylpiperazine(16)
4-Butanoyl-1-(4-fluorobenzoyl)piperazine (17)
4-Butanoyl-1-(4-fluorobenzensolfonyl)piperazine (18)
4-Pentanoyl-1-(4-fluorobenzensolfonyl)piperazine (19)
4-Hexanoyl-1-(4-fluorobenzensolfonyl)piperazine (20)
4-(4-methylbenzensolfonyl)-1,4-diazabicyclo[4.3.0]nonan-9-one (21)
4-(4-nitrobenzensolfonyl)-1,4-diazabicyclo[4.3.0]nonan-9-one (22)
4-(4-chlorobenzensolfonyl)-1,4-diazabicyclo[4.3.0]nonan-9-one (23)
4-(4-methoxybenzensolfonyl)-1,4-diazabicyclo[4.3.0]nonan-9-one (24)
4-(4-methylbenzoyl)-1,4-diazabicyclo[4.3.0]nonan-9-one (25)
4-Propionyl-1-(4-methylbenzensolfonyl)piperazine (26)
4-Propionyl-1-(4-methoxyylbenzensolfonyl)piperazine (27)
4-Propionyl-1-(4-chlorobenzensolfonyl)piperazine (28)
4-Propionyl-1-(4-nitrobenzensolfonyl)piperazine (29)
4-Propionyl-1-(4-methylbenzoyl)piperazine (30)
4-Propionyl-1-(4-methoxybenzoyl)piperazine (31)
4-Propionyl-1-(4-chlorobenzoyl)piperazine (32)
4-Propionyl-1-(4-nitrobenzoyl)piperazine (33).

4. Pharmaceutical compositions containing, as active component, a compound of formula (I) according to claim 1.

5. Use of the compounds claimed in claim 1 for the preparation of pharmaceutical compositions used to treat neurodegenerative pathologies.

6. Use, according to claim 5, wherein the neurodegenerative pathology is Alzheimer's disease.
